(19) Europäisches Patentamt
European Patent Office
Office européen des brevets

(11) Publication number: **0 194 864 B1**

(12) # EUROPEAN PATENT SPECIFICATION

(45) Date of publication of patent specification: **17.06.92**

(51) Int. Cl.5: **C07K 7/40**, A61K 37/26

(21) Application number: **86301755.4**

(22) Date of filing: **11.03.86**

The file contains technical information submitted after the application was filed and not included in this specification

(54) **Novel peptides.**

(30) Priority: **12.03.85 DK 1135/85**

(43) Date of publication of application:
**17.09.86 Bulletin 86/38**

(45) Publication of the grant of the patent:
**17.06.92 Bulletin 92/25**

(84) Designated Contracting States:
**AT BE CH DE FR GB IT LI LU NL SE**

(56) References cited:
**EP-A- 0 045 187**
**EP-A- 0 132 770**
**EP-A- 0 140 084**
**WO-A-86/05496**
**WO-A-86/05497**

**HOPPE-SEYLER'S Z. PHYSIOL. CHEM., vol. 360, November 1979, pages 1619-1632, Walter de Gruyter & Co., Berlin, DE; F. MÄRKI et al.: "Synthesis and biological activity of seventeen analogues of human insulin"**

(73) Proprietor: **NOVO NORDISK A/S**
**Novo Allé**
**DK-2880 Bagsvaerd(DK)**

(72) Inventor: **Markussen, Jan**
**7 Kikudbakken**
**DK-2730 Herlev(DK)**

(74) Representative: **Brown, John David et al**
**FORRESTER & BOEHMERT Franz-Joseph-Strasse 38**
**W-8000 München 40(DE)**

EP 0 194 864 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

BACKGROUND OF THIS INVENTION

The present invention relates to novel insulin compounds and to novel injectable solutions having prolonged insulin action.

In the treatment of diabetes mellitus, many varieties of insulin preparations have been suggested and used. Some of the preparations are fast acting and other preparations have more or less prolonged actions. Usually, pharmaceutical insulin preparations with more or less prolonged action are desirable. Such a prolonged action may be obtained by administering the insulin as a suspension of insulin crystals. The crystalline preparations can be obtained by crystallization of insulin in the presence of zinc (such as Lente™, see Schlichtkrull: Insulin Crystals, Chemical and Biological Studies on Insulin Crystals and Insulin Zinc Suspensions, Munksgaard, 1958) or by crystallization of insulin in the presence of zinc and protamine (such as NPH-insulin, see Rep.Steno Mem.Hosp. 1 (1946), 60).

One disadvantage in the use of the known suspensions of zinc insulin crystals or of zinc protamine insulin is the necessity of shaking the vial in order to ensure that the correct amount of insulin is being injected and to ensure that the concentration of insulin in the vial remains constant throughout its use. In Penfill™ cartridges where air must be absent, prolonged acting insulin suspensions require the incorporation of a solid body in the cartridge to enable agitation. The shaking of insulin suspensions and insulin solutions with air is in itself an undesirable process, as insulin has a tendency to denature under formation of fibrills at water-air interfaces. Consequently, solutions of insulins with prolonged action are desirable.

Solutions of insulin derivatives having a prolonged action was obtained from insulin that had been modified in its amino groups by reaction with phenylisocyanate (so-called Iso-insulin, see Hallas-Moeller: Chemical and Biological Insulin Studies based upon the Reaction between Insulin and Phenylisocyanate, Copenhagen 1945). Similarly, A1,B29-di-Boc substituted insulin (Boc designates tertiary butyloxycarbonyl) was reported to show a prolonged insulin action after subcutaneous administration (see Geiger & Enzmann in:

Proinsulin, Insulin, C-peptide; Proceedings of the Symposium on Proinsulin, Insulin and C-Peptide, Tokushima 1978; Amsterdam-Oxford 1979, 306 - 310). The A1,B29-di-Boc substituted insulin was found to exhibit a too slightly prolonged action to be clinically useful.

Solutions of unmodified insulins require large amounts of zinc ions (for example, 0.4 - 1 mg/U insulin) in order to exhibit a prolonged action (see J.Pharmacol. 55 (1935), 206). Injection of such large doses of zinc ions will probably cause pain and such solutions have, therefore, never been used in therapy.

The isoelectric point of insulin is about 5.5 and attempts have been made to decrease the solubility of insulin derivatives at neutral pH by shifting the isoelectric point upwards, for example, through additions, in the N-terminus of the B-chain, of basic amino acids like lysine or arginine (see, for example, German Offenlegungsschrift No. 2,042,299) or with the basic dipeptide arginyl-arginine (see Geiger & Enzmann cited above). The solubility of the latter compound, $Arg^{B(-1)}$-$Arg^{B0}$ insulin, near its isoelectric point was, however, much higher than that of the parent insulin.

Japanese patent application No. 55-144032 relates to analogues to human insulin wherein the B30-amino acid has been replaced by an amino acid having at least five carbon atoms, and amides and esters thereof. These insulin analogues were to be used in patients who had developed antibodies against mammalian insulins. In the Japanese patent application, six specific compounds are described, none of which were stated to have prolonged action. No specific injectable preparations are described in the Japanese patent application.

Hoppe-Seyler's Z. Physiol. Chem. (1979) 360 (11) pages 1619 to 1632 discloses seventeen analogues of human insulin, all of which produce the same biological effects as insulin but with markedly different potency.

EP-A-0045187 discloses a process for enzymatic replacement of the B30 amino acid of insulins and allows human insulin to be prepared from porcine insulin and threonine.

European patent application No. 84108442.9 relates to insulin analogues wherein a basic, organic group is attached to the B30-amino acid thereby introducing a positive charge at neutral pH. In these analogues, the B30-amino acid is neutral and, preferably, threonine as in human insulin. German patent application No. 3,327,709.5 relates to a suspension of crystals of the derivatives described in the above-noted European patent application as well as an aromatic hydroxy compound. German patent application No. 3,326,473.2 relates to a medicament containing a mixture of insulin compounds, of which at least one is described in the above-noted European patent application.

## BRIEF STATEMENT OF THE INVENTION

The present invention comprises novel analogs of human insulin that differ from human insulin by:

a) presence of an amide or ester residue on the C-terminal carboxyl group of the B-chain and

b) having at least one charge more than human insulin at pH 7, preferably not more than 4 charges more than human insulin at pH 7.

The change in charge is achieved by the blocking of the carboxylic group in the B30 amino acid and, if desired, by substituting one or more of the amino acids compared with human insulin.

In specific, the compounds of interest to practice of this invention are characterizable as follows: One or more of the four glutamic acid residues at A4, A17, B13, B21 is instead another naturally occurring neutral amino acid, preferably glutamine; and/or the threonine residue at B27 is instead a naturally occurring basic amino acid residue, preferably, L-arginine or L-lysine; and/or the threonine residue at B30 is instead one or two basic amino acid residues, one being preferred, and the N terminal carboxylic group in the B chain being protected.

The invention also comprises solutions of the above categorized human insulin analogs with a controlled level of zinc ions therein. The degree of prolongation of insulin action is enhanced and controlled thereby.

## DETAILED PRACTICE OF THIS INVENTION

It has surprisingly been found that injectable solutions with a combined short and prolonged insulin action can be made using, as the active ingredient, a single insulin derivative having the general formula I

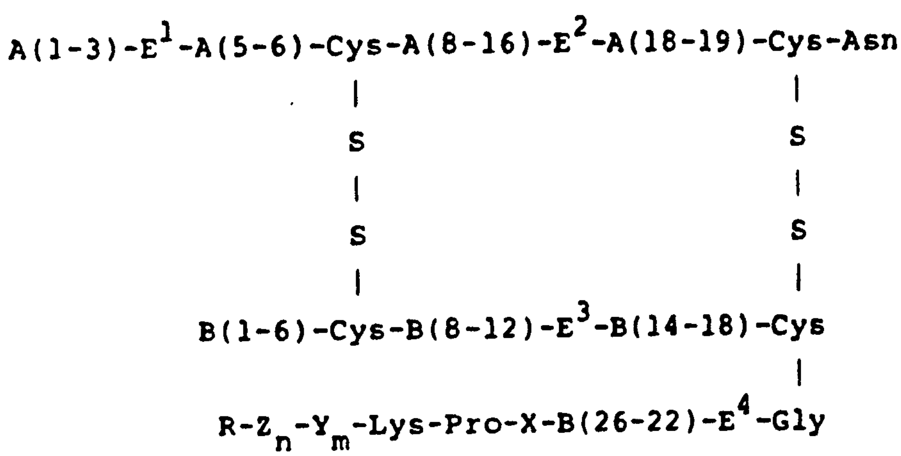

(A-chain)

(I)

(B-chain)

wherein the letters A and B followed by figures in parentheses designate the peptide fragments of the A- and B-chains, respectively, indicated by the figures in parentheses, $E^1$, $E^2$, $E^3$ and $E^4$ are the same or different each representing glutamic acid or a neutral amino acid residue which can be coded for by nucleotide sequences, X represents an L-threonine, L-arginine or L-lysine residue, Y and Z are the same or different and each represent an amino acid residue wherein any side chain amino group may be acylated and wherein any side chain hydroxy group may be alkylated, and m and n are the same or different and each represent zero or one, and R represents an amido or ester residue which blocks the C-terminal carboxyl group of the B-chain, with the proviso that not all of $E^1$, $E^2$, $E^3$ and $E^4$ are glutamic acid residues, when X is a threonine residue or, when $E^1$, $E^2$, $E^3$ and $E^4$ each is a glutamic acid residue, and X is a threonine residue, the group of formula $-Y_m-Z_n-R$ represents $-NH_2$, $-Arg-NH_2$,$-Arg-Arg-NH_2$, $-Arg-Lys-NH_2$, $-Dab-Dab-NH_2$, $-Dap-Dap-NH_2$, $-Lys-NH_2$, $-Lys(Lau)-NH_2$, $-Lys-Arg-NH_2$, $-Lys-Lys-NH_2$, $-Orn-NH_2$ and $-Orn-Orn-NH_2$.Lau designates lauroyl and Dab and Dap represents $\alpha,\gamma$-diaminobutyric acid and $\alpha,\beta$-diaminopropioic acid, respectively.

A subgroup of compounds of formula I is novel compounds having the general formula I wherein the letters A and B followed by figures in parentheses designate the peptide fragments of the A- and B-chains, respectively, indicated by the figures in parentheses, $E^1$, $E^2$, $E^3$ and $E^4$ are the same or different each representing glutamic acid or a neutral amino acid residue which can be coded for by nucleotide sequences, X represents an L-threonine, L-arginine or L-lysine residue, Y and Z are the same or different and each represent an amino acid residue wherein any side chain amino group may be acylated and wherein any side chain hydroxy group may be alkylated, and m and n are the same or different and each

represent zero or one, and R represents an amido or ester residue which blocks the C-terminal carboxyl group of the B-chain, with the proviso that not all of $E^1$, $E^2$, $E^3$ and $E^4$ are glutamic acid residues, when X is a threonine residue, or, when $E^1$, $E^2$, $E^3$ and $E^4$ each is a glutamic acid residue, and X is a threonine residue, the group of formula $-Y_m-Z_n-R$ represents $-NH_2$, $-Arg-NH_2$, $-Arg-Arg-NH_2$, $-Arg-Lys-NH_2$, $-Dab-Dab-NH_2$, $-Dap-Dap-NH_2$, $-Lys-NH_2$, $-Lys(Lau)-NH_2$, $-Lys-Arg-NH_2$, $-Lys-Lys-NH_2$, $-Orn-NH_2$ or $-Orn-Orn-NH_2$, with the further proviso that the compounds of formula I has at least one charge more than human insulin at a pH value of 7.

In compounds of formula I, the C-terminal carboxyl group of the B-chain is blocked by an ester group or amide group, thereby eliminating the negative charge of the carboxyl group. The change in charge introduced by the ester or amide group in position B30 can be further increased by substituting threonine in the B27-position with arginine or lysine and/or by substituting any of the four glutamic acid residues in the A4-, A17-, B13-, and B21-position with a neutral amino acid, preferably with a glutamine residue. Furthermore, a positive charge may be introduced by a basic amino acid in the B30- and/or B31-position. Since compounds of formula I can be applied in the clinic as solutions having a prolonged action, a decline in immunogenicity as compared to the commonly used suspensions of porcine or human insulins may occur.

The degree of prolongation can be enhanced and controlled by the addition of zinc ions.

Major parameters that control the degree of prolongation of the insulin effect are the concentration of zinc and the choice of the compound of formula I. With some analogs, e.g. $Arg^{B27}$,$Thr^{B30}-NH_2$ human insulin, very prolonged action is obtained with only 3 zinc atoms per hexamer unit of insulin analog corresponding to 8 $\mu$g zinc/ml in a preparation containing about 240 nmole/ml. With other analogs, e.g. $Lys^{B30}-NH_2$ human insulin, moderate prolongation of action is obtained with 30 zinc per hexamer of insulin analog corresponding to 80 $\mu$g zinc/ml in a preparation containing about 240 nmole/ml. The range for preferred zinc contrations extends from 0 to 2 mg/ml, preferably from 0 to 200 $\mu$g/ml zinc with substitution in the B13 and/or B27 position and preferably from 20 to 200 $\mu$g/ml with other analogs.

The prolonged action of solutions of compounds of formula I in the presence of zinc ions is ascribed to the low solubility of such compounds at neutral pH. Only solutions of insulin derivatives in which the C-terminal of the B-chain was blocked, showed a more prolonged action than Actrapid™ porcine insulin.

The pH of the injectable solution of this invention should preferably be below and so close to the physiological pH as possible, the upper limit being the pH where precipitation occurs. Stable solutions containing about 240 nmole/ml of compounds of formula I have been obtained at pH 5.5. The upper limit depends upon the constituents of the solution, i.e. isotonikum, preservative and zinc concentration, and upon the choice of compound of formula I. There is no lower pH limit of the solutions, but since the chemical stability of insulins is poor in acid solutions due to deamidation reactions and formation of dimers as high a pH as possible with respect to the physical stability of the solution is preferred. The preferred pH range for the injectable solutions of this invention is from 2.5 to 8.5, more preferred from 4.5 to 8.

A further aspect of this invention is that it provides improved flexibility for the patients. With two aqueous solutions, one containing a compound of formula I and the other containing a zinc salt, the patient can obtain a desired degree of prolonged action and a desired profile by mixing the two solutions appropriately. Thus, the patient has, using two stock solutions, the possibility of choosing one action and profile for the morning injection and another action and profile for the evening injection. Preferably, the zinc solution contains between about 10 $\mu$g and 20 mg zinc per ml. Alternatively, both of the stock solutions may contain zinc, either in the same or different concentrations, and/or both the stock solutions may contain a compound of formula I, either the same or different compounds.

Preferably, the injectable solutions of this invention have a strength of between about 60 and 6000 nmole/ml of the compound of formula I.

The neutral amino acid ($E^1$ through $E^4$) is, for example, glycine, valine, isoleucine, leucine, phenylalanine, tyrosine, methionine or preferably asparagine, glutamine, alanine, serine or threonine.

Examples of R are ester moieties, for example, lower alkoxy, preferably methoxy, ethoxy and most preferred tertiary butoxy, and such groups are present in compounds which are useful in the synthesis of human insulin, see, for example, U.S. Patent specification No. 4,343,898. Such esters are $Thr^{B30}-OBu^t$ human insulin and $Thr^{B30}(Bu^t)-OBu^t$ human insulin ($Bu^t$ designates tertiary butyl).

Furthermore, R can be a group of the general formula $-NR^1R^2$ wherein $R^1$ and $R^2$ are the same or different and each represents hydrogen or lower alkyl. Hereinafter the term "lower" designates that the group in question contains less than 7 carbon atoms, preferably less than 5 carbon atoms. An example of such a group is found in $Thr^{B30}-NH_2$ human insulin which is known as an intermediate in a synthesis of human insulin (see Carlsberg Res.Commun. 49 (1984), 463). In a preferred embodiment of this invention, R is $-NH_2$. Furthermore, R may be a lactam residue which preferably contains less than 8 atoms in the lactam

ring, for example a lactam of a diaminocarboxylic acid.

In a preferred embodiment of this invention, R is uncharged.

At neutral pH, the charge of $-X^{27}$ $-Pro^{28}-Lys^{29}-Y_m-Z_n-R$ is +1 in $Thr^{B30}-NH_2$ human insulin, $Thr^{B30}-OBu^t$ human insulin, $Thr^{B30}(Bu^t)-OBu^t$ human insulin and $Lys^{B29}-NH_2$,des-(B30) human insulin, +2 in $Lys^{B30}-NH_2$ human insulin, $Arg^{B30}-NH_2$ human insulin, $Orn^{B30}-NH_2$ human insulin, $Lys^{B27},Thr^{B30}-NH_2$ human insulin and $Arg^{B27},Thr^{B30}-NH_2$ human insulin, and +3 in $Lys^{B27},Lys^{B30}-NH_2$ human insulin, $Lys^{B27},Arg^{B30}-NH_2$ human insulin, $Arg^{B27},Lys^{B30}-NH_2$ human insulin, $Arg^{B27},Arg^{B30}-NH_2$ human insulin, $Lys^{B30}-Lys^{B31}-NH_2$ human insulin, $Arg^{B30}-Lys^{B31}-NH_2$ human insulin, $Arg^{B30}-Arg^{B31}-NH_2$ human insulin, $Orn^{B30}-Orn^{B31}-NH_2$ human insulin, $Dab^{B30}-Dab^{B31}-NH_2$ human insulin and $Dap^{B30}-Dap^{B31}-NH_2$ human insulin.

According to one preferred embodiment of this invention, the amino acid residues designated Y and Z are residues from L-amino acids which are coded for by nucleotide sequences.

Any side chain amino group in the amino acid residues designated Y and Z may be acylated by an acid containing from 2 to 18 carbon atoms, preferably a fatty acid containing from 6 to 18 carbon atoms, for example, lauric acid. Thus, $-Y_m-Z_n-R$ may be $-Lys(Lau)-NH_2$.

Examples of preferred alkylated hydroxy groups are methoxy, ethoxy and tertiary butoxy.

In one group of preferred compounds of formula I Y and/or Z is a basic amino acid residue wherein the side chain amino group optionally is acylated (m = 1).

In another group of preferred compounds of formula I n is zero and Y is a basic amino acid residue (m = 1).

In a further group of preferred compounds of formula I Y and Z are both basic amino acid residues (m = 1, n = 1).

Preferred compounds of formula I are each of the following: $Gln^{A17},Arg^{B27},Thr^{B30}-NH_2$ human insulin, $Gln^{A17},Gln^{B13},Thr^{B30}-NH_2$ human insulin, $Gln^{A17},Lys^{B27},Thr^{B30}-NH_2$ human insulin, $Gln^{A17},Lys^{B30}-NH_2$ human insulin, $Gln^{A17},Thr^{B30}-NH_2$ human insulin, $Gln^{B13},Arg^{B27},Thr^{B30}-NH_2$ human insulin, $Gln^{B13},Lys^{B27},Thr^{B30}-NH_2$ human insulin, $Gln^{B13},Lys^{B30}-NH_2$ human insulin, $Gln^{B13},Thr^{B30}-NH_2$ human insulin, $Arg^{B27},Arg^{B30}-NH_2$ human insulin, $Arg^{B27},Lys^{B30}-NH_2$ human insulin, $Arg^{B27},Thr^{B30}-NH_2$ human insulin, $Lys^{B27},Arg^{B30}-NH_2$ human insulin, $Lys^{B27},Lys^{B30}-NH_2$ human insulin, $Lys^{B27},Thr^{B30}-NH_2$ human insulin, $Lys^{B29}-NH_2$,des-(B30) human insulin, $Lys^{B30}-NH_2$ human insulin, $Lys^{B30}(Lau)-NH_2$ human insulin, $Lys^{B30}-Arg^{B31}-NH_2$ human insulin, $Lys^{B30}-Lys^{B31}-NH_2$ human insulin, $Arg^{B30}-NH_2$ human insulin, $Arg^{B30}-Arg^{B31}-NH_2$ human insulin or $Arg^{B30}-Lys^{B31}-NH_2$ human insulin.

Another preferred embodiment of this invention is preparations containing a compound of formula I wherein $E^1$, $E^2$, $E^3$ and/or $E^4$ is a glutamine residue, and/or X is Lys or Arg, and within this subclass of compounds of formula I, a further preferred embodiment is preparations containing a compound of formula I wherein the group $-Y_m-Z_n-R$ is $-Thr-NH_2$ or $-Lys-NH_2$. Preferred compounds are $Gln^{B13},Thr^{B30}-NH_2$ human insulin, $Gln^{A17},Thr^{B30}-NH_2$ human insulin, $Lys^{B27},Thr^{B30}-NH_2$ human insulin and $Arg^{B27},Thr^{B30}-NH_2$ human insulin.

In one group of preferred compounds of formula I, $E^1$, $E^3$ and $E^4$ is each a glutamic acid residue.

In another group of preferred compounds of formula I, $E^2$ is a glutamine residue.

In a still further group of preferred compounds of formula I, X is an arginine or lysine residue.

As is well known in the art, not all of the amino acid residues in human insulin are essential for the insulin action.

Indeed, porcine insulin and bovine insulin which differs from human insulin in amino acid residues have been employed to treat diabetics. Considerable species to species variations in the insulin molecule exist. Thus, many peptide residues in the human insulin molecule may be changed without undue diminution in insulin activity, including some peptide residues important to the isoelectric point of the molecule.

It is obvious that the groups designated $E^1$, $E^2$, $E^3$, $E^4$, X, Y, Z and R are to be selected so that the resulting compound of formula I is pharmaceutically acceptable.

In the known biphasic insulin preparations, it is common to combine fast acting, soluble insulin with prolonged acting, crystalline insulin in the same injection. Using compounds of formula I of this invention, a similar combined short and prolonged action can be obtained with a solution of a single compound of formula I. The ratio between fast and long effect decreases as the concentration of zinc ions in the solution is increased.

Compounds of formula I may be prepared by a transpeptidation reaction in which porcine insulin or else a biosynthetic precursor compound having the correct insulin disulphide bridges and having the general formula II:

$$B(1\text{-}12)\text{-}E^3\text{-}B(14\text{-}20)\text{-}E^4\text{-}B(22\text{-}26)\text{-}X\text{-}B(28\text{-}29)\text{-}(Q_q\text{-}R)_r\text{-}A(1\text{-}3)\text{-}E^1\text{-}A(5\text{-}16)\text{-}E^2\text{-}A(18\text{-}21) \quad (II)$$

wherein the letters A and B followed by figures in parentheses designate the appropriate peptide fragments of the A- and B-chains, respectively, as indicated by the figures in parentheses, Q is a peptide chain with q amino acids, q is an integer from 0 to 33, R is Lys or Arg, and r is zero or one, and $E^1$, $E^2$, $E^3$, $E^4$ and X each are as defined above, is reacted with an amino compound of the general formula III:

$H\text{-}Y_m\text{-}Z_n\text{-}R$ (III)

wherein Y, Z, R, m and n each are as defined above, and wherein side chain amino groups and hydroxy groups in Y and Z optionally are blocked with amino and hydroxy protecting groups, using trypsin or a trypsin like enzyme as a catalyst in a mixture of water and organic solvents as has been described in US Patent No. 4,343,898. Preferred compounds of formula III for use in this process are Thr-$NH_2$, Lys(Boc)-$NH_2$, Thr($Bu^t$)-$OBu^t$, Thr-$OBu^t$, Ala-$NH_2$ and Arg(Boc)-$NH_2$. Amino groups may be derivatized by acylation with a fatty acid. Hydroxy groups may be protected by alkylation. If Y and Z contain groups which are reversibly blocked by amino protecting groups, these groups may be removed at a later stage, if such is desired, after the amino protected intermediate has been separated from the trypsin or trypsin like enzyme. Of the trypsin like enzymes, lysyl endopeptidase from Achromobacter lyticus is useful.

The compound of formula II may be expressed in a host organism such as yeast similar to the description in EP-A-0163529 using a gene having the correct codons for the amino acids in question. The gene encoding the novel insulin derivative is then inserted into a suitable expression vector which when transferred to yeast is capable of expressing the desired compound. The product expressed is then isolated from the cells or the culture broth depending on whether it is secreted from the cells or not.

An example of a reversible amino protecting group is tertiary butoxycarbonyl and a reversible hydroxy protecting group is tertiary butyl. Such groups are removed under conditions which do not cause undesired alteration in the compound of formula I, for example, by trifluoroacetic acid.

Insulin compounds of formula I may also be prepared by a coupling reaction in which a compound of the general formula IV

```
A(1-3)-E1-A(5-6)-Cys-A(8-16)-E2-A(18-19)-Cys-Asn        (A-chain)
                  |                           |
                  S                           S
                  |                           |
                  S                           S              (IV)
                  |                           |
      B(1-6)-Cys-B(8-12)-E3-B(14-18)-Cys
                                              |              (B-chain)
             Lys-Pro-X-B(26-22)-E4-Gly
```

wherein $E^1$, $E^2$, $E^3$, $E^4$ and X each are as defined above, is coupled to an amino compound of the above formula III by trypsin or a trypsin like enzyme under conditions similar to those described in European patent specification No. 17,938.

When insulin is manufactured by genetic engineering the additional one or two positive charges may appropriately be introduced internally in the insulin molecule, i.e. in the A4-, A17-, B13-, B21- or B27-position, leaving for trypsin catalyzed semisynthesis blocking of the C-terminal carboxyl group of the B-chain with an amino acid amide or an amino acid ester.

The advantage in introducing the additional positive charges within the frame of the 51 amino acids of the insulin molecule to form the novel compounds of formula I rather than by prolongation of the B-chain beyond the 30 residues of the mammalian insulins relates to ease in preparation. In the semisynthetic transpeptidation a large molar excess of the amino acid amide or amino acid ester is employed. If a dipeptide amide or ester were to be used in the transpeptidation reaction, either price or solubility or both are prohibitive for use in large excess, and consequently the yield of the product becomes lower. Even when the same equimolar excess of, for example, Lys(Boc)-$NH_2$ and Lys(Boc)-Lys(Boc)-$NH_2$ is used in the transpeptidation reaction under similar conditions, the yield with the amino acid amide becomes substantially higher than with the dipeptide amide.

Insulin preparations of this invention are prepared by dissolving a compound of formula I in an aqueous medium at slightly acidic conditions, for example, in a concentration of 240 or 600 nmole/ml. The aqueous medium is made isotonic, for example, with sodium chloride or glycerol. Furthermore, the aqueous medium may contain zinc ions in a concentrations of up to about 20 µg of $Zn^{++}$ per unit of insulin activity, buffers such as acetate and citrate and preservatives such as m-cresol or phenol. The pH value of the solution is adjusted towards neutrality without getting too close to the isoelectric point of the compound of formula I in order to avoid precipitation. The pH value of the final insulin preparation depends upon the number of charges that have been changed in the compound of formula I, the concentration of zinc ions, the concentration of the compound of formula I and the compound of formula I selected. The insulin preparation is made sterile by sterile filtration.

The insulin preparations of this invention are used similarly to the use of the known insulin preparations.

Any novel feature or combination of features described herein is considered essential to this invention.

Herein the abbreviations used for the amino acids are those stated in J.Biol.Chem. 243 (1968), 3558. The amino acids stated herein are in L configuration. In formula I and elsewhere herein A(1-3) is Gly-Ile-Val, A(5-6) is Gln-Cys etc., cf. the amino acid sequence of human insulin. Unless otherwise indicated, the species of insulins stated herein is human.

Synthesis of the insulin compounds

The source of insulin was either porcine insulin or an insulin precursor expressed in yeast as described in EP-A-0163529.

The insulin precursors were recovered from the fermentation broths by adsorption to LiChroprep™ RP-18 as described in Example 7 of EP-A-0163529. The precursors were eluted from the column with 0.2 M KCl, 0.001 M HCl in 33% (v/v) ethanol. The insulin precursors were crystallized from the pool by successive additions of water (1 volume per volume of pool), solid trisodium citrate to make 0.05 M and finally zinc acetate to make 0.006 M. The pH was adjusted to 6.8 and the mixture was left overnight at 4°C. The crystals were isolated by centrifugaton, washed with water and dried in vacuo.

Protected amino acids and protected peptides for enzymatic semisynthesis were either prepared by standard methods or purchased (custom synthesis) from either Nova Biochem or Bachem, both Switzerland.

The letters TM after a name indicates that it is a trade mark.

Example 1

Synthesis of $Ly^{B30}$-$NH_2$ human insulin

Solutions of 1 g of porcine insulin dissolved in 4 ml of 7.5 M acetic acid and 6.1 g of Lys(Boc)-$HN_2$,$CH_3COOH$ (N epsilon-Boc-L-lysine amide, hydroacetate salt) dissolved to 15 ml with N,N-dimethylacetamide were mixed and the mixture was cooled to 12°C. A solution of 0.1 g of trypsin in 2.08 ml of a 0.05 M solution of calcium acetate was added. After 96 hours at 12°C, the proteins were precipitated by the addition of 200 ml of acetone, and the precipitate was isolated by centrifugation. The precipitate was washed once with 100 ml of acetone, isolated by centrifugation and dried in vacuo.

The precipitate was dissolved in 50 ml of 0.01 N hydrochloric acid in ethanol/water (28/72 parts per volume) and the solution was applied to a 5 x 30 cm preparative high pressure liquid chromatography (hereinafter designated HPLC) column packed with silica particles substituted with octadecyldimethylsilyl (mean particle size 15 micron, pore size 100 Ångstrøm). The column was equilibrated with ethanol/0.2 M solution of ammonium sulphate adjusted to pH 3.5 with sulphuric acid, in a ratio of 38/62 (parts per volume). The proteins were eluted from the column with the same buffer at a rate of 2 litre/h. $Lys(Boc)^{B30}$-$NH_2$ human insulin was found in a peak eluting from the column between 60 and 75 minutes, after elution of unreacted porcine insulin. The ethanol was evaporated in vacuo and the evaporation was continued until the volume was reduced to about 125 ml. The $Lys(Boc)^{B30}$-$NH_2$ human insulin was isolated by successive additions of 25 ml of acetone, 100 mg of citric acid (monohydrate p.a.) and 9 mg of zinc chloride (p.a.). The pH was adjusted to 6.5 and after 1 h at room temperature the crystallisation was continued at 4°C for 24 h with gentle stirring. The crystals were spun down, washed once with 5 ml of ice-cold water, spun down and dried in vacuo. Yield: 456 mg of $Lys(Boc)^{B30}$-$HN_2$ human insulin.

The $Lys(Boc)^{B30}$-$NH_2$ human insulin (456 mg) was dissolved in 15 ml of trifluoroacetic acid and left for 3 h at room temperature. The trifluoroacetic acid was removed by lyophilization. The lyophilisate was dissolved in 50 ml of water, the pH adjusted to 2.5 and 10 g of sodium chloride was added. The salt cake of

$Lys^{B30}$-$NH_2$ human insulin was isolated by centrifugation. The salt coke was dissolved in 125 ml of water and the $Lys^{B30}$-$NH_2$ human insulin was crystallized by successive additions of 25 ml of acetone, 100 mg of citric acid (monohydrate p.a.) and 9 mg of zinc chloride (p.a.) and adjustment of the pH to 7.0. After 1 h at room temperature, the crystallisation was continued at 4° C for 24 h with gentle stirring. The crystals were spun down, washed once with 5 ml of ice-cold water, spun down again and dried. Yield: 387 mg of crude $Lys^{B30}$-$NH_2$ human insulin.

The crystals were dissolved in 50 ml of 0.005 N hydrochloric acid in ethanol/water (20/80, parts per volume) and the solution was applied to a preparative HPLC column as described above, this time equilibrated with ethanol/0.3 M solution of potassium chloride and 0.001 N hydrochloric acid, in a ratio of 35.5/64.5 (parts per volume). Elution with the same buffer at a rate of 2 litres/h resulted in a peak of $Lys^{B30}$-$NH_2$ emerging from the column between 55 and 90 min. The products were isolated from the pool as described for $Lys(Boc)^{B30}$-$NH_2$ human insulin above, except that the pH in the crystallization in the zinc containing citrate buffer was adjusted to 7.0 rather than 6.5. Yield: 262 mg of pure $Lys^{B30}$-$NH_2$ human insulin.

The amino acid composition was in agreement with the theory, alanine being 1 residue/molecule and lysine being 2 residues/molecule. The product was pure in DISC PAGE electrophoresis at pH 8.9, the rate of migration being 55% of that of porcine insulin corresponding to a difference in charges of about 2. For details of the DISC PAGE electrophoresis see Horm.Metab.Res.Supplement Series No. 5 (1974), 134.

## Example 2

### Synthesis of $Arg^{B30}$-$NH_2$ human insulin and $Arg^{B30}$-$Arg^{B31}$-$NH_2$ human insulin

Solutions of 1 g of porcine insulin in 3.32 ml of 8 M acetic acid and 3.9 g of Arg-$NH_2$,$(CH_3COOH)_2$ (L-arginine amide dihydroacetate salt) dissolved to 10 ml with N,N-dimethylformamid (hereinafter designated DMF) were mixed, and the mixture was cooled to 12° C. A solution of 0.1 g of trypsin in 1.2 ml of a 0.05 M solution of calcium acetate was added. After 144 hours at 12° C, the proteins were precipitated by the addition of 200 ml of acetone, and the precipitate was isolated by centrifugation. The precipitate was washed once with 100 ml of acetone, isolated by centrifugation and dried in vacuo.

The precipitate was dissolved in 50 ml of 0.01 N hydrochloric acid in ethanol/water (27/73 parts per volume) and the proteins were applied to a preparative column as described in Example 1. At first, an eluent composed of ethanol/0.3 M solution of potassium chloride and 0.001 N hydrochloric acid in a ratio of 35/65 (parts per volume), was pumped through at a rate of 2 litres/h for 4 hours. Three unresolved peaks very recorded, from about 60 to 120 minutes, from 120 to 150 and from 150 to 180 minutes. The proteins in the three pools very isolated as described for $Lys^{B30}$-$NH_2$ in Example 1. Yields: 414 mg, 142 mg and 107 mg for pools I, II and III, respectively.

Amino acid analysis combined with DISC PAGE electrophoresis showed that the insulin molecules of pool I had been coupled with from 2 to several arginine residues. The major component of pool II was insulin coupled to a single arginine amide residue, i.e. $Arg^{B30}$-$NH_2$ human insulin. Pool III was a mixture of porcine insulin, des(B30) human insulin, $Arg^{B30}$ human insulin and $Arg^{B30}$-$NH_2$ human insulin.

### $Arg^{B30}$-$NH_2$ human insulin

The proteins of pool II was dissolved in 10 ml of ethanol/water 3/2 (v/v) at pH 2. After addition of 12 mg of EDTA the pH was raised to 10 by a 0.1 N solution of sodium hydroxide. The solution was applied to a 2.5 x 25 cm column of QAE-Sephadex A-25 equilibrated with a buffer composed of 0.5 M $NH_3$, 0.05 N hydrochloric acid and 0.04 M solution of sodium chloride in 60% ethanol (v/v). The column was eluted with 30 ml/h with a linear gradient in sodium chloride from 0.04 M to 0.1 M using a total of 1 litre of eluent, while the pH was kept constant at about 10.0. Fractions of 10 ml were collected. $Arg^{B30}$-$NH_2$ human insulin emerged from the columns in fractions Nos. 58 - 74. The product was isolated from the pool by evaporation followed by crystallization at pH 7 in a zinc containing citrate buffer containing 15% acetone (v/v) as described for $Lys^{B30}$-$NH_2$ in Example 1. Yield: 53 mg. The product was homogeneous in DISC PAGE electrophoresis at pH 8.9, the rate of migration being 55% of that of insulin. The amino acid composition was in accordance with the theory for $Arg^{B30}$-$NH_2$ human insulin, showing 2 arginine residues and 1 residue of alanine per molecule of insulin.

### $Arg^{B30}$-$Arg^{B31}$-$NH_2$ human insulin

The proteins of pool I were dissolved and subjected to ion exchange chromatography on QAE-Sephadex™ A-25 as described for the proteins in pool II. $Arg^{B30}$-$Arg^{B31}$-$NH_2$ human insulin emerged from the column in fractions Nos. 34 - 47. The product was isolated as described for $Lys^{B30}$-$NH_2$ human insulin in Example 1. Yield: 10 mg.

The product was homogeneous in DISC PAGE electrophoresis at pH 8.9, the rate of migration being 35% of that of insulin. The amino acid composition showed 3 arginine residues and 1 residue of alanine per molecule of insulin.

Example 3

Synthesis of $Gln^{A17}$,$Thr^{B30}$-$NH_2$ human insulin

To a suspension of 3.12 g of $Gln^{A17}$,B(1-29)-Ala-Ala-Lys-A(1-21) insulin precursor in 15 ml of acetic acid/DMF/water (11.4 ml acetic acid, 65.1 ml DMF, water to make 100 ml) 30 ml of 1 M Thr-$NH_2$ in DMF was added. The mixture was cooled to 12°C and 0.3 g of porcine trypsin dissolved in 7.5 ml of 0.05 M calcium acetate was added. Stirring was continued until the insulin precursor had dissolved. After 48 hours at 12°C the proteins were precipitated by addition of 400 ml of acetone. The proteins were isolated by centrifugation, washed once with 100 ml of acetone and dried in vacuo.

The precipitate was dissolved in 70 ml of 0.04 N HCl, the pH adjusted to 2.5 and the derivative was purified by HPLC as described in Example 1, except that an eluent composed of 35 parts of ethanol and 65 parts of 0.3 M KCl, 0.001 N HCl was used for elution. The derivative emerged from the column after about 3 column volums, and it was isolated by successive additions of 1 volume of water, solid trisodium citrate to make 0.05 M and solid zinc acetate to make 0.006 M. After adjustment of pH to 6.5 and stirring overnight at 4°C, crystals were harvested by centrifugation, washed with water and dried. Yield 1.64 g = 53%. Further purifications by anion exchange chromatography as described for human insulin esters (see Markussen, ibid, 410). Final yield: 1.15 g = 37%. The product was near homogeneous in DISC PAGE electrophoresis at pH 8.9, the rate of migration being 55% of that of insulin. In analytical reverse-phase HPLC (see Markussen ibid, 410) the product elutes at about the same rate as porcine insulin. The purity found to about 95%. Amino acid composition analysis showed identity to the suggested formula.

Example 4

Synthesis of $Gln^{A17}$,$Lys^{B30}$-$NH_2$ human insulin

To a suspension of 3.15 g of $Gln^{A17}$,B(1-29)-Ala-Ala-Lys-A(1-21) insulin precursor in 15 ml of acetic acid/DMF/water (4.57 ml acetic acid, 71.9 ml of DMF, water to make 100 ml) 30 ml of 0.4 M Lys(Boc)-$NH_2$ in DMF was added. The mixture was cooled to 12°C and 0.3 g of trypsin dissolved in 7.5 ml of 0.05 M calcium acetate was added. Stirring was continued until the insulin precursor had dissolved. After 48 hours at 12°C the proteins were isolated as described in Example 3.

The precipitate was dissolved in 70 ml of 0.04 N HCl, the pH adjusted to 2.5 and $Gln^{A17}$,$Lys(Boc)^{B30}$-$NH_2$ human insulin was purified by HPLC as described in Example 1, except that elution was performed first with 2.3 l of an eluent composed of 37 parts ethanol and 63 parts of 0.3 M KCl, 0.001 N HCl, followed by an eluent composed of 39 parts of ethanol and 61 parts of aqueous 0.3 M KCl, 0.001 N HCl. The derivate emerged 25 minutes after the change of eluent, and it was isolated as described for $Gln^{A17}$,$Thr^{B30}$-$NH_2$ human insulin in Example 3. Yield of $Gln^{A17}$,$Lys(Boc)^{B30}$-$NH_2$ human insulin 906 mg = 29%.

$Gln^{A17}$,$Lys(Boc)^{B30}$-$NH_2$ human insulin (1.55 g) was dissolved in 30 ml of trifluoroacetic acid (TFA) and left at room temperature for 2 hours. The TFA was removed by lyophilization. The residue was dissolved in 15 ml of water, the pH adjusted to 3 with 1 N NaOH and 22 ml of ethanol was added. The solution was applied to a 2.5 x 20 cm column of SP-Sephadex™ C-25 equilibrated with an ethanol/water 3/2 (v/v) buffer comprising 0.01 M citric acid, 0.03 M NaCl, pH adjusted to 4.5 with NaOH. The column was eluted with the same buffer, using a linear gradient in NaCl from 0.03 M to 0.4 M in a total of 1.6 l of eluent. The derivative eluted in 440 ml when the gradient reached 0.2 M NaCl. It was crystallized by addition of 1100 ml of water, solid trisodium citrate to make 0.05 M and solid zinc acetate to make 0.006 M. Eventually the pH was adjusted to 6.8. After stirring overnight at 4°C the crystals were isolated by centrifugation, washed once with water and dried in vacuo. Yield: 1.00 g corresponding to 65% over last step and 19% from the $Gln^{A17}$,B(1-29)-Ala-Ala-Lys-A(1-21) insulin precursor. The product was near homogeneous in DISC PAGE electrophoresis at pH 8.9, the rate of migration being 35% of that of insulin. In analytical HPLC the product emerges before porcine insulin, the purity being about 97%. Amino acid composition analysis showed 2

lysine residues per molecule, and otherwise identity to human insulin.

Example 5

Synthesis of $Arg^{B27}$,$Thr^{B30}$-$NH_2$ human insulin

To a suspension of 3.8 g of $Arg^{B27}$,B(1-29)-Ala-Ala-Lys-A(1-21) insulin precursor in 18 ml of acetic acid/water/DMF (11.4 ml acetic acid, 35 ml water, DMF to make 100 ml) 36 ml of 1 M Thr-$NH_2$ in DMF was added. The mixture was cooled to 12°C and 0.38 g of porcine trypsin in 6.84 ml of 0.05 M calcium acetate was added. After 48 hours at 12°C the proteins were precipitated with acetone as described in Example 3.

The derivative was purified by HPLC as described in Example 1 using first 1800 ml eluent composed of 35 parts of ethanol and 65 parts of aqueous 0.3 M KCl, 0.001 N HCl, followed by an eluent composed of 37 parts of ethanol and 63 parts of the aqueous solution. The derivative emerged 10 minutes after shift in eluent and it was isolated as described for $Gln^{A17}$,$Thr^{B30}$-$NH_2$ insulin in Example 3. Finally it was purified on a column of SP-Sephadex™ C-25 as described for $Gln^{A17}$,$Lys^{B30}$-$NH_2$ human insulin in Example 4.

The yield of $Arg^{B27}$,$Thr^{B30}$-$NH_2$ human insulin was 1.63 g corresponding to 43%. Essentially one band was seen in DISC PAGE electrophoresis, the rate of migrating being 55% of that of insulin. In analytical HPLC the product emerge before porcine insulin, the purity being about 96%. Amino acid composition analysis shows 2 arginine residues per molecule, and otherwise identity to human insulin.

Example 6

Synthesis of $Arg^{B27}$,$Lys^{B30}$-$NH_2$ human insulin

The compound was synthesized from 3.61 g of $Arg^{B27}$,B(1-29)-Ala-Ala-Lys-A(1-21) insulin precursor using the methods described in Example 4. Yield of $Arg^{B27}$,$Lys^{B30}$-$NH_2$ human insulin 0.78 g = 22%. One major band in DISC PAGE electrophoresis migrating 35% of the distance of insulin migration. Two minor bands visible. Purity in analytical HPLC 92%; the product emerge before porcine insulin. Amino acids composition analysis shows 2 arginine and 2 lysine residues per molecule and otherwise identity to human insulin.

Example 7

Synthesis of $Lys^{B27}$,$Thr^{B30}$-$NH_2$ human insulin

The compound was synthesized from 7.0 g of $Lys^{B27}$,B(1-29)-Ala-Ala-Lys-A(1-21) insulin precursor using the methods described in Example 5. Yield of $Lys^{B27}$,$Thr^{B30}$-$NH_2$ human insulin was 3.15 g corresponding to 45%. DISC PAGE electrophoresis showed one major band and two minor bands, the main band migrating 55% of the distance of the insulin reference band. The purity in analytical HPLC was 96%. The compound emerge earlier than porcine insulin in reverse phase HPLC. Amino acid composition analysis shows 2 lysine residues per molecule and otherwise identity to human insulin.

Example 8

Synthesis of $Lys^{B27}$,$Lys^{B30}$-$NH_2$ human insulin

The compound was synthesized from 7.0 g of $Lys^{B27}$,B(1-29)-Ala-Ala-Lys-A(1-21) insulin precursor using the methods described in Example 4. Yield of $Lys^{B27}$,$Lys^{B30}$-$NH_2$ human insulin was 1.57 g corresponding to 22%. DISC PAGE electrophoresis showed one major band migrating to a distance of 35% of that of porcine insulin. One minor impurity is visible. Purity in analytical HPLC was 94%, the compound eluting well ahead of porcine insulin. Amino acid composition analysis showed 3 lysine residues per molecule and otherwise identity to human insulin.

Example 9

Synthesis of $Gln^{B13}$,$Thr^{B30}$-$NH_2$ human insulin

The compound was synthesized from 3.05 g of $Gln^{B13}$,B(1-29)-Ala-Ala-Lys-A(1-21) insulin precursor

using the methods described in Example 5. Yield of final product was 0.88 g corresponding to 29%. DISC PAGE electrophoresis showed one major band, migrating 55% of the distance porcine insulin migrates. Purity by HPLC was 95%, the compound eluting later than porcine insulin. Amino acid composition analysis showed identity to that of human insulin.

Example 10

Preparation of injectable solutions of compounds of formula I

Sterile injectable solutions of the compounds of formula I for testing of the degree of prolonged action were made using 0.9% (w/v) sodium chloride as the isotonicum and 0.15% (v/v) m-cresol as the preservative. Injectable solutions were also made using 1.6% (w/v) glycerol as the isotonicum, using 0.3% (w/v) m-cresol as the preservative, and being buffered with 0.01 M sodium acetate. The concentration of zinc ions was varied from 0 to 160 μg/ml. The pH values of the solutions were adjusted sufficiently off the isoelectric point of the compounds of formula I to keep the solutions clear upon storage at 4° C. The solutions contained 240 nmole/ml of the compounds of formula I. The concentration of 240 nmole/ml was established by measurement of the absorbance at 276 nm of a more concentrated stock solution devoid of m-cresol, using the molar extinction coefficient for porcine insulin of 6100 for these derivatives (see Handbuch der Inneren Medizin, Vol. 7/Part 2A, Editor: Oberdisse, 1975, 113) and using the established potency for monocomponent porcine insulin of 28.5 U/mg dry substance (see Diabetes Care, Vol. 6/Supplement 1 (1983), 4). 1 U corresponds to 5.95 nmole.

Injectable solutions containing 240 nmole/ml of the compounds of formula I stated in Table 1 and having the pH values and content of zinc stated in the table were made.

Test for prolongation of insulin effect

The prolongation of the hypoglycemic effect produced by the injectable solutions of insulin was tested according to British Pharmacopoeia 1980, A 142, in fasted rabbits. Each test solution was administered subcutaneously in a dosis of 15.5 nmole per rabbit in 6 or 12 animals weighing 3 - 4 kg, and the course of the hypoglycemia was followed for 6 hours. For comparison the fast acting preparation, Actrapid™ porcine insulin, was included in the tests. The results of the tests are shown in Table 1 and 2.

The result of tests for prolonged effect of certain compounds in rabbits is stated in Table 1, below. The glucose value is the mean, from 6 rabbits, of the glucose value in per cent of the initial value. Solutions were made isotonic with 0.9% NaCl, using 0.15% (v/v) m-cresol as the preservative.

Table 1

| Compound of formula 1 | $Zn^{++}$, μg/ml | pH | Glucose in percent of initial | | | | |
|---|---|---|---|---|---|---|---|
| | | | 1/2 h | 1 h | 2 h | 4 h | 6 h |
| $Lys^{B30}$-$NH_2$ insulin | 0 | 4.5 | 53 | 54 | 49 | 79 | 100 |
| $Lys^{B30}$-$NH_2$ insulin | 80 | 4.5 | 63 | 62 | 63 | 73 | 80 |
| $Lys^{B30}$-$NH_2$ insulin | 160 | 4.5 | 87 | 80 | 68 | 90 | 93 |
| $Arg^{B30}$-$NH_2$ insulin | 0 | 4.5 | 57 | 53 | 51 | 65 | 81 |
| $Arg^{B30}$-$NH_2$ insulin | 80 | 4.5 | 76 | 68 | 63 | 73 | 78 |
| $Thr^{B30}$-$NH_2$ insulin | 0 | 4.2 | 46 | 46 | 39 | 64 | 91 |
| $Thr^{B30}$-$NH_2$ insulin | 160 | 4.2 | 64 | 58 | 50 | 70 | 69 |
| $Thr^{B30}$-$OBu^t$ insulin | 0 | 4.0 | 59 | 65 | 59 | 79 | 100 |
| $Thr^{B30}$-$OBu^t$ insulin | 160 | 4.0 | 81 | 75 | 62 | 66 | 88 |
| $Thr^{B30}(Bu^t)$-$OBu^t$ insulin | 0 | 4.0 | 64 | 60 | 54 | 71 | 82 |
| $Arg^{B30}$-$Arg^{B31}$-$NH_2$ insulin | 0 | 4.5 | 72 | 70 | 68 | 67 | 68 |
| $Arg^{B30}$-$Arg^{B31}$-$NH_2$ insulin | 160 | 4.5 | 91 | 87 | 78 | 73 | 68 |
| Actrapid™ porcine insulin | 15 | 7 | 53 | 48 | 42 | 70 | 98 |

The result of tests for prolonged effect of certain compounds in rabbits is stated in table 2, below. The glucose value is the mean, from 12 rabbits, of the glucose value in percent of the initial value. Test solutions were made isotonic with 1.6% (w/v) glycerol, using 0.3% (w/v) m-cresol as the preservative, and being

buffered by 0.01 M sodium acetate.

Table 2

| Compound of formula I | $Zn^{++}$, µg/ml | pH | Glucose in percent of initial 1 h | 2 h | 4 h | 6 h |
|---|---|---|---|---|---|---|
| $Gln^{A17}$,$Thr^{B30}$-$NH_2$ insulin | 80 | 4.5 | 65 | 63 | 68 | 83 |
| $Gln^{A17}$,$Lys^{B30}$-$NH_2$ insulin | 80 | 4.5 | 60 | 56 | 73 | 86 |
| $Gln^{B13}$,$Thr^{B30}$-$NH_2$ insulin | 6.7 | 4.5 | 91 | 92 | 92 | 90 |
| $Arg^{B27}$,$Thr^{B30}$-$NH_2$ insulin | 80 | 4.5 | 88 | 86 | 85 | 81 |
| $Arg^{B27}$,$Thr^{B30}$-$NH_2$ insulin | 8.5 | 4.5 | 62 | 64 | 66 | 67 |
| $Arg^{B27}$,$Lys^{B30}$-$NH_2$ insulin | 80 | 4.5 | 85 | 83 | 81 | 79 |
| $Arg^{B27}$,$Lys^{B30}$-$NH_2$ insulin | 10.9 | 4.5 | 78 | 73 | 69 | 67 |
| $Lys^{B27}$,$Thr^{B30}$-$NH_2$ insulin | 7.4 | 4.5 | 56 | 55 | 62 | 61 |
| $Lys^{B27}$,$Lys^{B30}$-$NH_2$ insulin | 9.5 | 4.5 | 72 | 65 | 65 | 60 |
| $Lys^{B30}$-$NH_2$ insulin | 80 | 4.5 | 74 | 83 | 80 | 82 |
| Reference insulin | | | | | | |
| Actrapid™ porcine insulin | 15 | 7 | 58 | 56 | 87 | 100 |

The potencies of insulin compounds were assessed in the mouse blood sugar depletion test (British Pharmacopoeia 1980, A 141 - A 142). In order to minimize the problem of estimating potency of insulins having a timing different from the standard, insulin solutions for potency determinations were made up without additions of zinc. Solutions were made up to contain 240 nmole/ml based on the absorbance at 276 nm. The zinc content of solutions were 8 - 10 µg/ml, arizing from the crystalline derivatives. The estimated potencies of some insulin compounds are shown in Table 3, below.

Table 3

| | Potency relative to insulin, % | Confidence limits (P = 0.05), % |
|---|---|---|
| $Gln^{A17}$,$Thr^{B30}$-$NH_2$ insulin | 67 | 58 - 75 |
| $Gln^{A17}$,$Lys^{B30}$-$NH_2$ insulin | 62 | 51 - 72 |
| $Arg^{B27}$,$Thr^{B30}$-$NH_2$ insulin | 122 | 102 - 145 |
| $Arg^{B27}$,$Lys^{B30}$-$NH_2$ insulin | 84 | 74 - 94 |

## Claims

**1.** Compounds of the general formula I

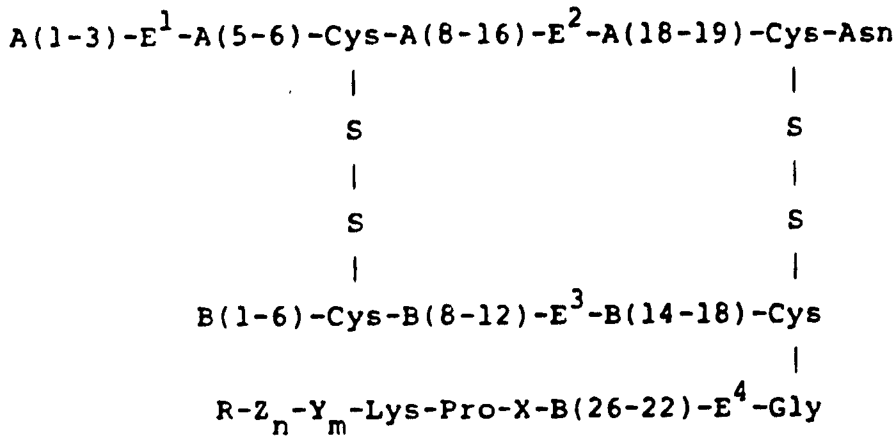

(A-chain)

(I)

(B-chain)

wherein the letters A and B followed by figures in parentheses designate the peptide fragments of the A- and B-chains, respectively, indicated by the figures in parentheses, $E^1$, $E^2$, $E^3$ and $E^4$ are the same or different each representing glutamic acid or a neutral amino acid residue which can be coded for by nucleotide sequences, X represents an L-threonine, L-arginine or L-lysine residue, Y and Z are the same or different and each represent an amino acid residue wherein any side chain amino group may be acylated and wherein any side chain hydroxy group may be alkylated, and m and n are the same or different and each represent zero or one, and R represents an amido or ester residue which blocks the C-terminal carboxyl group of the B-chain, with the proviso that not all of $E^1$, $E^2$, $E^3$ and $E^4$ are glutamic acid residues, when X is a threonine residue, or, when $E^1$, $E^2$, $E^3$ and $E^4$ each is a glutamic acid residue, and X is a threonine residue, the group of formula $-Y_m-Z_n-R$ represents $-NH_2$, $-Arg-NH_2$, $-Arg-Arg-NH_2$, $-Arg-Lys-NH_2$, $-Dab-Dab-NH_2$, $-Dap-Dap-NH_2$, $-Lys-NH_2$, $-Lys(Lau)-NH_2$, $-Lys-Arg-NH_2$, $-Lys-Lys-NH_2$, $-Orn-NH_2$ or $-Orn-Orn-NH_2$, with the further proviso that the compounds of formula I has at least one charge more than human insulin at a pH value of 7.

**2.** Compound according to Claim 1, characterized in that $E^1$, $E^3$ and $E^4$ each is a glutamic acid residue.

**3.** Compounds according to any one of the preceding claims, characterized in that $E^2$ is a glutamine residue.

**4.** Compounds according to any one of the preceding claims, characterized in that Y and/or Z is a basic amino acid residue wherein the side chain amino group optionally is acylated (m = 1).

**5.** Compounds according to any one of the preceding claims, characterized in that n is zero, and Y is a basic amino acid residue (m = 1).

**6.** Compounds according to any one of the preceding claims, characterized in that Y and Z are both basic amino acid residues (m = 1, n = 1).

**7.** Compounds according to any one of the preceding claims, characterized in that R is a group of the general formula $-N^1R^2$ wherein $R^1$ and $R^2$ are the same or different and each represent hydrogen or lower alkyl, and preferably R is $-NH_2$.

**8.** Compounds according to any one of the Claims 1 through 6, characterized in that R is lower (containing less than 7 carbon atoms) alkoxy, preferably tertiary butyloxy.

**9.** Compounds according to any one of the Claims 1 through 6, characterized in that R is a residue of a lactam which preferably contains less than 8 atoms in the lactam ring.

**10.** Compound according to Claim 1, characterized in that it is $Gln^{A17}$,$Arg^{B27}$,$Thr^{B30}-NH_2$ human insulin, $Gln^{A17}$,$Gln^{B13}$,$Thr^{B30}-NH_2$ human insulin, $Gln^{A17}$,$Lys^{B27}$,$Thr^{B30}-NH_2$ human insulin, $Gln^{A17}$,$Lys^{B30}-NH_2$

human insulin, $Gln^{A17}$,$Thr^{B30}$-$NH_2$ human insulin, $Gln^{B13}$,$Arg^{B27}$,$Thr^{B30}$-$NH_2$ human insulin, $Gln^{B13}$,$Lys^{B27}$,$Thr^{B30}$-$NH_2$ human insulin, $Gln^{B13}$,$Lys^{B30}$,-$NH_2$ human insulin, $Gln^{B13}$,$Thr^{B30}$-$NH_2$ human insulin, $Arg^{B27}$,$Arg^{B30}$-$NH_2$ human insulin, $Arg^{B27}$,$Lys^{B30}$-$NH_2$ human insulin, $Arg^{B27}$,$Thr^{B30}$-$NH_2$ human insulin, $Lys^{B27}$,$Arg^{B30}$-$NH_2$ human insulin, $Lys^{B27}$,$Lys^{B30}$-$NH_2$ human insulin, $Lys^{B27}$,$Thr^{B30}$-$NH_2$ human insulin, $Lys^{B29}$-$NH_2$,des-(B30) human insulin, $Lys^{B30}$-$NH_2$ human insulin, $Lys^{B30}$(Lau)-$NH_2$ human insulin, $Lys^{B30}$-$Arg^{B31}$-$NH_2$ human insulin, $Lys^{B30}$-$Lys^{B31}$-$NH_2$ human insulin, $Arg^{B30}$-$NH_2$ human insulin, $Arg^{B30}$-$Arg^{B31}$-$NH_2$ human insulin or $Arg^{B30}$-$Lys^{B31}$-$NH_2$ human insulin.

**11.** Injectable solutions with prolonged insulin action, characterized in that it contains a compound in accordance with Claim 1.

**12.** Preparation according to Claim 11, characterized in that it contains zinc ions, preferably from about 2 μg to about 2 mg zinc per ml, most prefered from about 5 μg to 200 μg zinc per ml.

**13.** Zinc solution for use in preparing a more prolonged acting solution according to Claim 1 by mixing it with a solution containing a compound of formula I stated in Claim 1, which zinc solution preferably contains between about 10 μg and 20 mg zinc per ml.

**14.** A method for preparing a solution according to Claim 11, characterized in that a solution of a compound of formula I stated in Claim 1, optionally containing zinc, is mixed with a zinc solution optionally containing a compound of formula I stated in Claim 1, rendering a mixture with a zinc concentration of up to 2 mg/ml, and preferably the content of zinc in each of the two solutions is less than about 4 mg/ml.

**15.** A process for preparing compounds of the general formula I stated in Claim 1, characterized in a) transpeptidating porcine insulin or a compound of the general formula II:

$$B(1\text{-}12)\text{-}E^3\text{-}B(14\text{-}20)\text{-}E^4\text{-}B(22\text{-}26)\text{-}X\text{-}B(28\text{-}29)\text{-}(Q_q\text{-}R)_r\text{-}A(1\text{-}3)\text{-}E^1\text{-}A(5\text{-}16)\text{-}E^2\text{-}A(18\text{-}21) \quad (II)$$

wherein A and B designate the fragments of the A- and B-chains indicated by numbers in parentheses, Q is a peptide chain with q amino acids, q is an integer from 0 to 33, R is Lys or Arg, and r is zero or one and $E^1$, $E^2$, $E^3$, $E^4$ and X each are as defined in Claim 1, with a compound of the general formula III:

$$H\text{-}Y_m\text{-}Z_n\text{-}R \quad (III)$$

where Y, Z, R, m and n each are as defined above, and wherein side chain amino groups and hydroxy groups in Y and Z optionally are blocked with amino and hydroxy protecting groups, using trypsin or a trypsin like enzyme as a catalyst, or b) coupling a compound of formula IV

```
A(1-3)-E¹-A(5-6)-Cys-A(8-16)-E²-A(18-19)-Cys-Asn        (A-chain)
                  |                       |
                  S                       S
                  |                       |
                  S                       S                (IV)
                  |                       |
       B(1-6)-Cys-B(8-12)-E³-B(14-18)-Cys
                                          |                (B-chain)
                   Lys-Pro-X-B(26-22)-E⁴-Gly
```

wherein $E^1$, $E^2$, $E^3$, $E^4$ and X each are as defined in Claim 11, with a compound of formula III by trypsin or a trypsin like enzyme.

## Revendications

1. Composés de la formule générale I

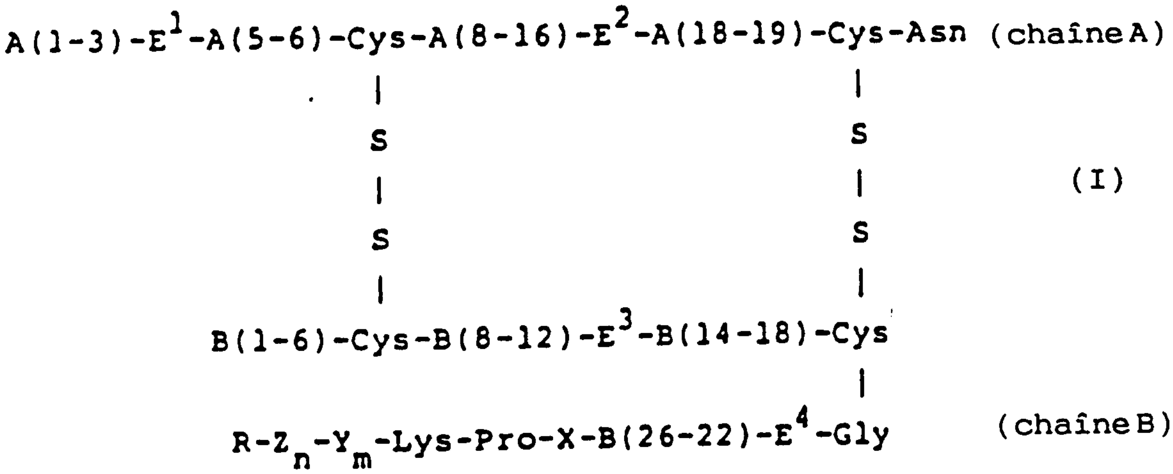

dans laquelle les lettres A et B suivies des chiffres entre parenthèses désignent les fragments de peptide des chaînes A et B, respectivement, désignées par les chiffres entre parenthèses, $E^1$, $E^2$, $E^3$ et $E^4$ sont identiques ou différents, chacun représentant l'acide glutamique ou un reste d'acide aminé neutre qui peut être codé par des séquences nucléotides, X représente un reste de L-thréonine, de L-arginine ou de L-lysine, Y et Z sont identiques ou différents et chacun représente un reste d'acide aminé dans lequel tout groupe aminé de chaîne latérale peut être acylé et dans lequel tout groupe hydroxy de chaîne latérale peut être alkylé, et m et n sont identiques ou différents et chacun représente zéro ou un, et R représente un reste amide ou ester qui bloque le groupe carboxyle en terminaison C de la chaîne B, sous réserve que la totalité de $E^1$, $E^2$, $E^3$ et $E^4$ ne sont pas des restes d'acide glutamique, lorsque X est un reste de thréonine ou lorsque $E^1$, $E^2$, $E^3$ et $E^4$ sont chacun un reste d'acide glutamique, et X est un reste de thréonine, le groupe de formule $-Y_m-Z_n-R$ représente $-NH_2$, $-Arg-NH_2$, $-Arg-Arg-NH_2$,$-Arg-Lys-NH_2$, $-Dab-Dab-HN_2$, $Dap-Dap-NH_2$, $-Lys-NH_2$, $-Lys(Lau)-NH_2$, $-Lys-Arg-NH_2$, $-Lys-Lys-NH_2$, $-Orn-NH_2$ ou $-Orn-Orn-NH_2$,à la condition supplémentaire que les composés de formule I possèdent au moins une charge de plus que l'insuline humaine à une valeur de pH de 7.

2. Composé selon la revendication 1, caractérisé en ce que $E^1$, $E^3$ et $E^4$ représentent chacun un reste d'acide glutamique.

3. Composés selon l'une quelconque des revendications précédentes, caractérisés en ce que $E^2$ est un reste de glutamine.

4. Composés selon l'une quelconque des revendications précédentes, caractérisés en ce que Y et/ou Z sont un reste d'acide aminé basique dans lequel le groupe aminé de chaîne latérale est facultativement acylé ($m = 1$).

5. Composés selon l'une quelconque des revendications précédentes, caractérisés en ce que n est égal à zéro, et Y est un reste d'acide aminé basique ($m = 1$).

6. Composés selon l'une quelconque des revendications précédentes, caractérisés en ce que Y et Z sont tous deux des restes d'acide aminé basique ($m = 1$, $n = 1$).

7. Composés selon l'une quelconque des revendications précédentes, caractérisés en ce que R est un groupe de formule générale $-NR^1R^2$ dans lequel $R^1$ et $R^2$ sont identiques ou différents et chacun représente l'hydrogène ou un alkyle inférieur, et de préférence R est $-NH_2$.

8. Composés selon l'une quelconque des revendications 1 jusqu'à 6, caractérisés en ce que R est un

alcoxy inférieur contenant moins de 7 atomes de carbone, de préférence un butyloxy tertiaire.

**9.** Composés selon l'une quelconque des revendications 1 à 6, caractérisés en ce que R est un reste de lactame qui contient de préférence moins de 8 atomes dans le cycle lactam.

**10.** Composé selon la revendication 1, caractérisé en ce qu'il s'agit de $Gln^{A17}$, $Arg^{B27}$, $Thr^{B30}$-$NH_2$ insuline humaine, $Gln^{A17}$, $Gln^{B13}$, $Thr^{B30}$-$NH_2$ insuline humaine, $Gln^{A17}$, $Lys^{B27}$, $Thr^{B30}$-$NH_2$ insuline humaine, $Gln^{A17}$, $Lys^{B30}$-$NH_2$insuline humaine, $Gln^{A17}$, $Thr^{B30}$-$NH_2$ insuline humaine, $Gln^{B13}$, $Arg^{B27}$, $Thr^{B30}$-$NH_2$ insuline humaine $Gln^{B13}$, $Lys^{B27}$, $Thr^{B30}$-$NH_2$ insuline humaine, $Gln^{B13}$, $Lys^{B30}$-$NH_2$ insuline humaine, $Gln^{B13}$, $Thr^{B30}$-$NH_2$ insuline humaine, $Arg^{B27}$, $Arg^{B30}$-$NH_2$ insuline humaine, $Arg^{B27}$, $Lys^{B30}$-$NH_2$ insuline humaine, $Arg^{B27}$, $Thr^{B30}$-$NH_2$ insuline humaine, $Lys^{B27}$, $Arg^{B30}$-$NH_2$ insuline humaine, $Lys^{B27}$, $Lys^{B30}$-$NH_2$ insuline humaine, $Lys^{B27}$, $Thr^{B30}$-$NH_2$ insuline humaine, $Lys^{B29}$-$NH_2$ des-(B30) insuline humaine, $Lys^{B30}$-$NH_2$ insuline humaine, $Lys^{B30}$(Lau)-$NH_2$ insuline humaine, $Lys^{B30}$-$Arg^{B31}$-$NH_2$ insuline humaine, $Lys^{B30}$-$Lys^{B31}$-$NH_2$ insuline humaine, $Arg^{B30}$-$NH_2$ insuline humaine, $Arg^{B30}$-$Arg^{B31}$-$NH_2$ insuline humaine ou $Arg^{B30}$-$Lys^{B31}$-$NH_2$ insuline humaine.

**11.** Solution injectable avec action d'insuline prolongée, caractérisée en ce qu'elle contient un composé selon la revendication 1.

**12.** Préparation selon la revendication 11, caractérisée en ce qu'elle contient des ions de zinc, de préférence d'environ 2 $\mu$g jusqu'à environ 2 mg de zinc par ml, de façon plus préférée d'environ 5 $\mu$g jusqu'à 200 $\mu$g de zinc par ml.

**13.** Solution de zinc destinée à l'utilisation dans la préparation d'une solution à action plus prolongée selon la revendication 1 en la mélangeant avec une solution contenant un composé de formule I énoncée dans la revendication 11, laquelle solution de zinc contient de préférence entre environ 10 $\mu$g et 20 mg de zinc par ml.

**14.** Procédé destiné à la préparation d'une solution selon la revendication 11, caractérisé en ce qu une solution d'un composé de la formule I énoncée à la revendication 1, contenant facultativement du zinc, est mélangée avec une solution de zinc contenant facultativement un composé de la formule I énoncée à la revendication 1, rendant un mélange avec une concentration de zinc jusqu'à 2 mg/ml, et de préférence la teneur en zinc dans chacune des deux solutions est inférieure à environ 4 mg/ml.

**15.** Procédé destiné à la préparation de composés de la formule générale I énoncée dans la revendication 1, caractérisé en ce que a) l'on transpeptidate de l'insuline porcine ou un composé de la formule générale II :

$$\mathbf{B(1\text{-}12)\text{-}E^3\text{-}B(14\text{-}20)\text{-}E^4\text{-}B(22\text{-}26)\text{-}X\text{-}B(28\text{-}29)\text{-}(Q_q\text{-}R)_r\text{-}A(1\text{-}3)\text{-}E^1\text{-}A(5\text{-}16)\text{-}E^2\text{-}A(18\text{-}21)} \quad \mathbf{(II)}$$

dans laquelle A et B désignent les fragments des chaînes A et B indiqués par les chiffres entre parenthèses, Q est une chaîne peptide avec des acides aminés q, q est un nombre entier de 0 à 33, R est Lys ou Arg et r est zéro ou un et $E^1$, $E^2$, $E^3$, $E^4$ et X sont chacun tels que définis dans la revendication 2 avec un composé de la formule générale III:

$$H\text{-}Y_m\text{-}Z_n\text{-}R \quad (III)$$

où Y, Z, R, m et n sont chacun tels que définis ci-dessus et dans laquelle les groupes aminés de chaîne latérale et les groupes hydroxy en Y et Z sont facultativement bloqués avec des groupes protecteurs hydroxy et amino, en utilisant la trypsine ou une enzyme similaire à la trypsine en tant que catalyseur, ou b) en couplant un composé de formule IV

```
A(1-3)-E1-A(5-6)-Cys-A(8-16)-E2-A(18-19)-Cys-Asn (chaîne A)
                |                        |
                S                        S
                |                        |               (IV)
                S                        S
                |                        |
       B(1-6)-Cys-B(8-12)-E3-B(14-18)-Cys
                                         |          (chaîne B)
                Lys-Pro-X-B(26-22)-E4-Gly
```

dans laquelle $E^1$, $E^2$, $E^3$, $E^4$ et X sont chacun tels que définis dans la revendication 11 avec un composé de formule III par la trypsine ou une enzyme similaire à la trypsine.

## Patentansprüche

1. Verbindungen der allgemeinen Formel I

```
A(1-3)-E1-A(5-6)-Cys-A(8-16)-E2-A(18-19)-Cys-Asn
                 |                         |        (A-Kette)
                 S                         S
                 |                         |            (I)
                 S                         S
                 |                         |        (B-Kette)
        B(1-6)-Cys-B(8-12)-E3-B(14-18)-Cys
                                           |
         R-Zn-Ym-Lys-Pro-X-B(26-22)-E4-Gly
```

wobei die Buchstaben A und B, gefolgt von Zahlen in Klammern, die Peptidfragmente der A- bzw. B-Ketten bezeichnen, angegeben durch die Zahlen in Klammern, $E^1$, $E^2$, $E^3$ und $E^4$ identisch oder verschieden sind, wobei jedes Glutaminsäure oder einen neutralen Aminosäurerest darstellt, der durch Nukleotidsequenzen kodiert werden kann, X einen L-Threonin-, L-Arginin- oder L-Lysin-Rest darstellt, Y und Z identisch oder verschieden sind und jedes einen Aminosäurerest darstellt, bei dem jede Seitenketten-Aminogruppe acyliert sein kann und bei dem jede Seitenketten-Hydroxygruppe alkyliert sein kann, und m und n identisch oder verschieden sind und jedes 0 oder 1 darstellt und R einen Amido- oder Esterrest darstellt, der die C-terminale Carboxylgruppe der B-Kette blockiert, mit der Maßgabe, daß nicht alle Gruppen $E^1$, $E^2$, $E^3$ und $E^4$ Glutaminsäurereste sind, wenn X ein Threoninrest ist, oder, wenn $E^1$, $E^2$, $E^3$ und $E^4$ alle ein Glutaminsäurerest sind und X ein Threoninrest ist, die Gruppe der Formel $-Y_m-Z_n-R-NH_2$, $-Arg-NH_2$, $-Arg-Arg-NH_2$, $-Arg-Lys-NH_2$, $-Dab-Dab-NH_2$, $-Dap-Dap-NH_2$, $-Lys-NH_2$, $-Lys(Lau)-NH_2$, $-Lys-Arg-NH_2$, $-Lys-Lys-NH_2$, $-Orn-NH_2$ oder $-Orn-Orn-NH_2$ darstellt, mit der weiteren Maßgabe, daß die Verbindungen der Formel I wenigstens eine Ladung mehr als Humaninsulin bei einem pH-Wert von 7 besitzen.

2. Verbindung nach Anspruch 1, dadurch gekennzeichnet, daß $E^1$, $E^3$ und $E^4$ alle ein Glutaminsäurerest sind.

3. Verbindung nach einem der vorangehenden Ansprüche, dadurch gekennzeichnet, daß $E^2$ ein Glutaminrest ist.

4. Verbindung nach einem der vorangehenden Ansprüche, dadurch gekennzeichnet, daß Y und/oder Z ein basischer Aminosäurerest ist, bei dem die Seitenketten-Aminogruppe fakultativ acyliert ist (m = 1).

5. Verbindung nach einem der vorangehenden Ansprüche, dadurch gekennzeichnet, daß n Null ist und Y ein basischer Aminosäurerest ist (m = 1).

6. Verbindungen nach einem der vorangehenden Ansprüche, dadurch gekennzeichnet, daß sowohl Y als auch Z basische Aminosäurereste sind (m = 1, n = 1).

7. Verbindung nach einem der vorangehenden Ansprüche, dadurch gekennzeichnet, daß R eine Gruppe der allgemeinen Formel - $NR^1R^2$ ist, wobei $R^1$ und $R^2$ identisch oder verschieden sind und jedes Wasserstoff oder niederes Alkyl darstellt und R vorzugsweise $-NH_2$ ist.

8. Verbindung nach einem der Ansprüche 1 bis 6, dadurch gekennzeichnet, daß R niederes (weniger als 7 Kohlenstoffatome enthaltendes) Alkoxy, vorzugsweise tertiäres Butyloxy ist.

9. Verbindung nach einem der Ansprüche 1 bis 6, dadurch gekennzeichnet, das R ein Rest eines Lactams ist, der vorzugsweise weniger als 8 Atome im Lactamring enthält.

10. Verbindung nach Anspruch 1, dadurch gekennzeichnet, daß sie $Gln^{A17}$, $Arg^{B27}$, $Thr^{B30}$-$NH_2$-Humaninsulin, $Gln^{A17}$, $Gln^{B13}$, $Thr^{B30}$-$NH_2$-Humaninsulin, $Gln^{A17}$, $Lys^{B27}$, $Thr^{B30}$-$NH_2$-Humaninulin, $Gln^{A17}$, $Lys^{B30}$-$NH_2$-Humaninsulin, $Gln^{A17}$, $Thr^{B30}$-$NH_2$-Humaninsulin, $Gln^{B13}$, $Arg^{B27}$, $Thr^{B30}$-$NH_2$-Humaninsulin, $Gln^{B13}$, $Lys^{B27}$, $Thr^{B30}$-$NH_2$-Humaninsulin, $Gln^{B13}$, $Lys^{B30}$-$NH_2$-Humaninsulin, $Gln^{B13}$, $Thr^{B30}$-$NH_2$-Humaninsulin, $Arg^{B27}$, $Arg^{B30}$-$NH_2$-Humaninsulin, $Arg^{B27}$, $Lys^{B30}$-$NH_2$-Humaninsulin, $Arg^{B27}$, $Thr^{B30}$-$NH_2$-Humaninsulin, $Lys^{B27}$, $Arg^{B30}$-$NH_2$-Humaninsulin, $Lys^{B27}$, $Lys^{B30}$-$NH_2$-Humaninsulin, $Lys^{B27}$, $Thr^{B30}$-$NH_2$-Humaninsulin, $Lys^{B29}$-$NH_2$, des-(B30)-Humaninsulin, $Lys^{B30}$-$NH_2$-Humaninsulin, $Lys^{B30}$(Lau)-$NH_2$-Humaninsulin, $Lys^{B30}$-$Arg^{B31}$-$NH_2$-Humaninsulin, $Lys^{B30}$-$Lys^{B31}$-$NH_2$-Humaninsulin, $Arg^{B30}$-$NH_2$-Humaninsulin, $Arg^{B30}$-$Arg^{B31}$-$NH_2$-Humaninsulin oder $Arg^{B30}$-$Lys^{B31}$-$NH_2$-Humaninsulin ist.

11. Injizierbare Lösungen mit verlängerter Insulinwirkung, dadurch gekennzeichnet, daß sie eine Verbindung nach Anspruch 1 enthält.

12. Zubereitung nach Anspruch 11, dadurch gekennzeichnet, daß sie Zink-Ionen enthält, vorzugsweise von etwa 2 $\mu$g bis etwa 2 mg Zink pro ml, am bevorzugtesten von etwa 5 $\mu$g bis 200 $\mu$g Zink pro ml.

13. Zinklösung zur Verwendung bei der Herstellung einer Lösung mit verlängerter Wirkung gemäß Anspruch 1, indem sie mit einer Lösung vermischt wird, die eine Verbindung der Formel I, angegeben in Anspruch 1, enthält, wobei diese Zinklösung vorzugsweise zwischen etwa 10 $\mu$g und 20 mg Zink pro ml enthält.

14. Verfahren zur Herstellung einer Lösung nach Anspruch 11, dadurch gekennzeichnet, daß eine Lösung einer Verbindung der Formel I, angegeben in Anspruch 1, die fakultativ Zink enthält, mit einer Zinklösung vermischt wird, die fakultativ eine Verbindung der Formel I, angegeben in Anspruch 1, enthält, was eine Mischung mit einer Zinkkonzentration von bis zu 2 mg/ml liefert, und daß der Zinkgehalt in jeder der zwei Lösungen geringer ist als etwa 4 mg/ml.

15. Verfahren zur Herstellung von Verbindungen der allgemeinen Formel I, angegeben in Anspruch 1, dadurch gekennzeichnet, daß a) Schweineinsulin oder eine Verbindung der allgemeinen Formel II:

$$B(1\text{-}12)\text{-}E^3\text{-}B(14\text{-}20)\text{-}E^4\text{-}B(22\text{-}26)\text{-}X\text{-}B(28\text{-}29)\text{-}(Q_q\text{-}R)_r\text{-}A(1\text{-}3)\text{-}E^1\text{-}A(5\text{-}16)\text{-}E^2\text{-}A(18\text{-}21) \quad (II)$$

wobei A und B die Fragmente der A- und B-Ketten bezeichnen, angegeben durch Zahlen in Klammern, Q eine Peptidkette mit q Aminosäuren ist, q eine ganze Zahl von 0 bis 33 ist, R Lys oder Arg ist und r Null oder Eins ist und $E^1$, $E^2$, $E^3$, $E^4$ und X jeweils wie in Anspruch 1 definiert sind, mit einer Verbindung der allgemeinen Formel III:

H-$Y_m$-$Z_n$-R (III)

in der Y, Z, R, m und n jeweils wie oben definiert sind und wobei Seitenketten-Aminogruppen und -Hydroxygruppen in Y und Z fakultativ mit Amino- und Hydroxy-Schutzgruppen blockiert sind, unter Verwendung von Trypsin oder einem trypsinähnlichen Enzym als Katalysator, transpeptidiert wird, oder b) eine Verbindung der Formel IV:

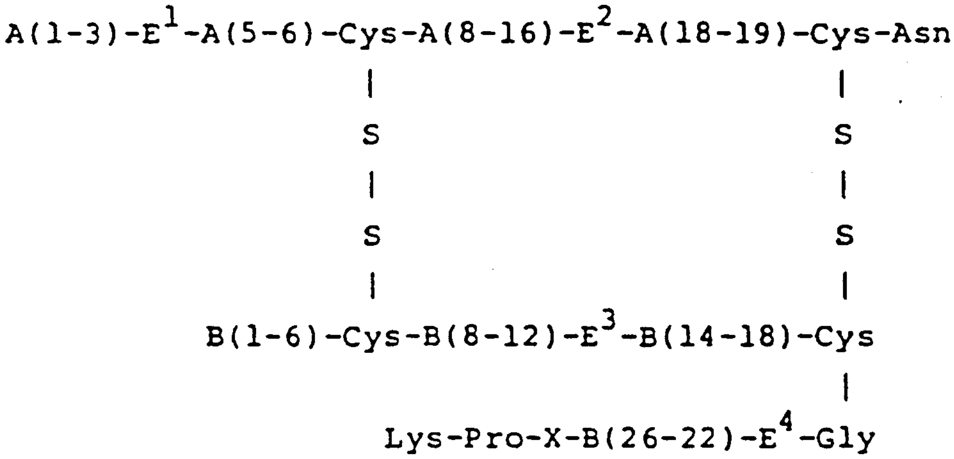

(A-Kette)

(IV)

(B-Kette)

wobei $E^1$, $E^2$, $E^3$, $E^4$ und X jeweils wie in Anspruch 1 definiert sind, durch Trypsin oder ein trypsinähnliches Enzym mit einer Verbindung der Formel III gekoppelt wird.